# DEMANDE DE BREVET EUROPEEN

(11) **EP 4 570 217 A1**
(43) Date de publication de la demande: **18.06.2025**
(21) Numéro de dépôt: 24219672.3
(22) Date de dépôt: 13.12.2024
(51) Int. Cl.: A61F 5/44, A61F 5/451

(54) **SAC HYGIÉNIQUE À USAGE MÉDICAL**

(30) Priorité: 15.12.2023 FR 2314270
(71) Demandeur: Cleanis, 94110 Arcueil (FR)
(72) Inventeur: ALFONSO, Inès, 38130 ECHIROLLES (FR)
(74) Mandataire: Cabinet Nony

(57) **Abrégé**

Sac hygiénique (10) à usage médical destiné à la récupération de déchets produits par un patient, ledit sac hygiénique (10) comportant :
- une enveloppe (12) étanche pourvue d'une ouverture (20), l'enveloppe (12) étant formée d'un film en matière plastique (29),
- optionnellement, au moins un lien de fermeture (14₁, 14₂) de ladite ouverture, le lien de fermeture (14₁, 14₂) étant notamment monté coulissant dans un ourlet (24₁, 24₂) de l'enveloppe (12) de manière qu'une extraction dudit lien de fermeture (14₁, 14₂) hors dudit ourlet (24₁, 24₂) réduise ladite ouverture (20),
caractérisé en ce que le film en matière plastique (29) de l'enveloppe comporte plus de 30% de plastique recyclé.

## Description

### Domaine technique

L'invention concerne un sac hygiénique comportant du plastique recyclé destiné à la récupération de déchets, notamment de déchets liquides, produits par un patient, notamment dans les hôpitaux. L'invention concerne aussi un procédé de fabrication d'un tel sac hygiénique. L'invention concerne enfin une bande de tels sacs hygiéniques et un ensemble comportant de tels sacs hygiéniques, ainsi qu'un emballage desdits sacs hygiéniques.

### Technique antérieure

Dans les hôpitaux, il peut être nécessaire de récupérer les déchets produits par certains patients (urine, excréments, vomissures) parce que ces patients ne peuvent se déplacer aux toilettes, ou à des fins d'analyse. A cet effet, les patients peuvent avoir recours à des sacs hygiéniques, pouvant être disposés, selon les besoins, sur un support, comme un bassin de lit, un seau de chaise percée ou des WC. Un tel sac hygiénique est par exemple décrit dans le brevet FR 3 081 103.

Les sacs hygiéniques connus sont principalement composés de matière plastique vierge. Dans l'optique d'être plus respectueux de l'environnement, des industriels ont mené des recherches pour réduire la part de plastique vierge au sein de ces sacs. Ce processus est néanmoins complexe. Remplacer une partie du plastique vierge par des matières organiques, comme de l'amidon de maïs s'est ainsi avéré impossible en raison, d'une part, de la trop rapide dégradation des matières organiques qui limite la durée de vie des sacs hygiéniques, et, d'autre part, des faibles performances mécaniques des sacs hygiéniques résultant de cette combinaison, qui sont par exemple plus sensibles aux frottements ou marques d'ongles, les rendant susceptibles aux trous. La garantie d'un sac hermétique est en effet fondamentale dans le cadre médical pour empêcher la prolifération des bactéries provenant des déchets. Ces matières organiques ont enfin un approvisionnement limité, rendant leur utilisation pour une production de masse difficile.

Il a été proposé de remplacer une petite partie du plastique vierge utilisé dans un sac par du plastique recyclé post-industriel, aussi dénommé plastique PIR, de « *Post Industrial Recycling* » en anglais. Le plastique PIR est un plastique fabriqué au moyen d'une résine plastique recyclée produite à partir de chutes de production de plastique vierge dans des usines. Cependant, il n'a été possible de n'utiliser qu'une faible part de plastique recyclé PIR au sein des sacs, le reste étant du plastique vierge.

Bien que fonctionnelle d'un point de vue mécanique, cette option reste cependant limitée d'un point de vue environnemental. Dans l'état de la technique actuelle, augmenter la proportion de plastique recyclé dans les sacs hygiéniques est toutefois difficile. En effet, au-delà d'une faible proportion de plastique recyclé au sein d'un sac, la matière recyclée utilisée pour produire la résine nécessaire à la fabrication des sacs est jugée instable car il est difficile de garantir une traçabilité et une répétabilité des déchets plastiques la composant. Par ailleurs, augmenter la proportion de plastique recyclé rend difficile le maintien de la couleur blanche du produit, indispensable pour un usage médical car synonyme d'hygiène et de stérilité.

Il existe donc un besoin pour bénéficier d'un sac hygiénique disposant de bonnes performances mécaniques pour être utilisé dans un cadre médical, et qui comporte suffisamment de plastique recyclé.

### Résumé de l'invention

L'invention vise à répondre à ce besoin et a ainsi pour objet un sac hygiénique à usage médical destiné à la récupération de déchets produits par un patient, ledit sac hygiénique comportant :
- une enveloppe étanche pourvue d'une ouverture, l'enveloppe étant formée d'un film en matière plastique,
- optionnellement, au moins un lien de fermeture de ladite ouverture, le lien de fermeture étant notamment monté coulissant dans un ourlet de l'enveloppe de manière qu'une extraction dudit lien de fermeture hors dudit ourlet réduise ladite ouverture,
caractérisé en ce que le film en matière plastique de l'enveloppe comportant plus de 30% de plastique recyclé.

Par « à usage médical », on entend que le sac hygiénique est un dispositif médical, qui peut entrer en contact avec la peau d'un patient. Il doit ainsi se conformer au Règlement des Dispositifs Médicaux (MDR) et à la norme ISO 13485.

Les déchets produits par le patient peuvent être au moins en partie liquides et/ou au moins en partie solides. Il peut s'agir d'urine, d'excréments, et/ou de vomissures, notamment.

Le sac hygiénique peut être un protège seau et/ou un protège bassin. Le sac hygiénique peut en variante être un protège-WC. En variante, il peut s'agir d'un sac urinal ou d'un sac vomitoire.

L'enveloppe du sac hygiénique peut contenir un volume de déchets compris entre 200 et 1000 mL, voire entre 400 et 800 mL, par exemple 450 mL ou 500 mL ou encore 700 mL.

Le sac hygiénique peut être un sac antimicrobien.

L'enveloppe peut comporter des premier et deuxième feuillets identiques, superposés l'un à l'autre et fixés l'un à l'autre le long de bords latéraux droits et gauche et d'un bord de fond.

L'enveloppe peut être dépourvue de soufflet. L'enveloppe peut être dépourvue de bretelles de préhension.

Le film en matière plastique de l'enveloppe peut être d'une épaisseur inférieure à 40 microns, notamment comprise entre 30 et 36 microns, par exemple d'environ 32 microns. Diminuer l'épaisseur du film plastique en dessous de l'épaisseur « standard » de 40 microns permet de réduire l'utilisation de matière plastique.

Le film en matière plastique peut être en polyéthylène (PE).

Dans un mode de réalisation, le sac hygiénique peut comporter au moins un lien de fermeture de ladite ouverture, le lien de fermeture étant notamment monté coulissant dans un ourlet de l'enveloppe de manière qu'une extraction dudit lien de fermeture hors dudit ourlet réduise ladite ouverture.

Le ou les liens de fermetures peuvent être des rubans. Chaque ruban peut présenter une largeur supérieure à 10 mm, mieux supérieure à 15 mm, encore mieux supérieure à 20 mm, de préférence d'environ 25 mm.

Dans un mode de réalisation où le sac hygiénique est un sac urinal, le ou les liens de fermeture peuvent être des cordelettes. Chaque cordelette peut présenter une largeur supérieure à 2 mm, mieux supérieure à 5 mm, encore mieux supérieure à 10 mm, de préférence supérieure à 15 mm.

Le ou les liens de fermeture peuvent être plastique, notamment en polyéthylène

Le plastique recyclé peut être fabriqué à partir d'une résine plastique recyclée de grade suffisamment élevé pour que l'indice de fluidité à chaud de la résine soit supérieur ou égal à 1,40 g/10min, mieux supérieur ou égal à 1,50g/10min, encore mieux supérieur ou égal à 1,60g/10 min et/ou pour que la densité de la résine soit supérieure à 0,9g/cm³.

Le plastique recyclé peut être fabriqué à partir d'une résine plastique recyclée de grade suffisamment élevé pour que l'indice de fluidité à chaud de la résine soit supérieur ou égal à 1,40 g/10min, mieux supérieur ou égal à 1,50g/10min, encore mieux supérieur ou égal à 1,60g/10 min. L'indice de fluidité à chaud peut être mesurée selon la norme ASTMD1238.

Le plastique recyclé peut être fabriqué à partir d'une résine plastique recyclée de grade suffisamment élevé pour que la densité de la résine soit supérieure à 0,9g/cm³. La densité peut être mesurée selon la norme ASTMD792.

Le grade NAT% PLUS est un grade utilisé par la société CY Plastique, sur une échelle comprenant notamment, du grade le plus faible au grade plus élevé, les grades suivants : N1, N1A, N1B, N2, N3, AB, AA, AAA, NAT, NAT%, NAT% PLUS. Ce grade NAT% PLUS correspond à une résine présentant une densité d'environ 0,9169g/cm³ et d'indice de fluidité à chaud d'environ 1,60g/10 min. La résine plastique recyclée utilisée peut être de grade supérieur ou égal au grade NAT% PLUS.

Le grade d'une résine est un indicateur de sa qualité, dépendant de son niveau de contamination par des éléments externes comme la terre, la poussière, l'huile, les produits chimiques ou les engrais par exemple. La qualité de la résine est ainsi intrinsèquement liée aux déchets plastiques sélectionnés pour la produire. Une résine de haute qualité résulte d'un processus de tri élevé et d'une sélection de déchets plastiques peu contaminés pour la produire. Les résines de différents grades peuvent se distinguer par leur couleur, témoignant de leur niveau de contamination. Les résines de grade élevé sont ainsi plus claires que les résines de faible grade.

Le grade d'une résine a un impact sur le niveau des impuretés produites au cours de la formation du film plastique ainsi que sur ses performances mécaniques, notamment sa résistance aux perforations. En effet plus la résine contient d'impuretés et plus son grade est faible, plus le risque de formation de trous dans le film plastique est élevé, notamment lors du processus d'extrusion du plastique.

En choisissant une résine de grade élevé pour produire le film plastique, on peut ainsi augmenter le pourcentage de plastique recyclé dans le sac hygiénique tout en conservant les performances mécaniques nécessaires à son utilisation dans un cadre médical.

Le film en matière plastique de l'enveloppe peut comporter plus de 30% de plastique recyclé post-consommation (PCR), mieux au moins 40% de plastique PCR, mieux au moins 50% de plastique PCR, encore mieux au moins 60% de plastique PCR, encore mieux au moins 70% de plastique PCR, encore mieux au moins 80% de plastique PCR, encore mieux au moins 90% de plastique PCR, notamment 100% de plastique PCR.

Le plastique recyclé post-consommation, dit plastique PCR, pour « *Post Consumer Recycled* » en anglais, est fabriqué au moyen d'une résine plastique recyclée produite à partir de déchets plastiques utilisés quotidiennement par des utilisateurs, comme les sacs plastiques, les emballages de supermarchés, les films de palettes, cette liste n'étant pas limitative.

Le volume de plastique PCR disponible est beaucoup plus important que le volume de plastique recyclé post-industriel (PIR) disponible. Par ailleurs, pour un groupe industriel, opter pour du plastique PCR contribue plus fortement à aider le groupe à réduire son empreinte carbone, à diminuer son impact sur les décharges et à atteindre ses objectifs globaux en matière de développement durable.

Le film en matière plastique de l'enveloppe peut comporter plus de 30% de plastique recyclé post-industriel (PIR), mieux au moins 40% de plastique PIR, mieux au moins 50% de plastique PIR, encore mieux au moins 60% de plastique PIR, encore mieux au moins 70% de plastique PIR, encore mieux au moins 80% de plastique PIR, encore mieux au moins 90% de plastique PIR, notamment 100% de plastique PIR.

Le plastique recyclé post-industriel est un plastique considéré comme relativement propre et uniforme, et peu susceptible de contenir des impuretés car il n'a pas été souillé par une utilisation extérieure. Le plastique recyclé post-industriel est facilement traçable et susceptible de répondre aux exigences de la réglementation MDR et de la norme ISO 13485.

Le film en matière plastique de l'enveloppe peut comporter strictement moins de 100% de plastique recyclé post-industriel (PIR), voire moins de 90%, mieux moins de 80%, voire encore moins de 70%, mieux encore moins de 60%, voire moins de 50%, notamment moins de 40%, voire moins de 30% de plastique recyclé post-industriel (PIR), mieux moins de 20%, voire moins de 10%, notamment 0% de plastique PIR. Le film en matière plastique de l'enveloppe peut être dépourvu de plastique recyclé post-industriel (PIR).

Le film en matière plastique peut comporter au moins 30% de polyéthylène à basse densité (LDPE), mieux au moins 50%, voire au moins 70%, notamment 100% de polyéthylène à basse densité.

Le polyéthylène à basse densité dit LDPE, pour « *low density polyethylen* » en anglais, peut être préféré au polyéthylène à haute densité (HDPE) ou au polyéthylène à basse densité linéaire (LLDPE).

Le film en matière plastique peut notamment comporter moins de 30 % de polyéthylène à haute densité, mieux moins de 20 %, voire moins de 10 %, notamment 0% de polyéthylène à haute densité (HDPE). Le film en matière plastique de l'enveloppe peut être dépourvu de polyéthylène à haute densité (HDPE). Le LDPE est moins cassant que le HDPE. Il se froisse moins facilement et est moins bruyant quand on le touche, d'où son avantage pour une utilisation dans un cadre médical.

Le film en matière plastique peut comporter moins de 30 % de polyéthylène à basse densité linéaire (LLPDE), mieux moins de 20 %, voire moins de 10 %, notamment 0% de polyéthylène à basse densité linéaire. Le film en matière plastique de l'enveloppe peut être dépourvu de polyéthylène à basse densité linéaire (LLPDE). Contrairement au LLDPE, le LDPE ne nécessite pas l'ajout d'un additif pour le rendre plus collant lors de sa fabrication. En utilisant du LDPE, il y ainsi moins de risques d'observer des marques de colle dans le film produit, pouvant rendre l'enveloppe du sac difficile à ouvrir et entraînant un mauvais coulissement du ou des liens de fermeture dans l'ourlet de l'enveloppe.

La résine plastique recyclée peut être configurée pour que le film en matière plastique soit de couleur blanche lorsqu'observé en lumière blanche et/ou ait un aspect brillant. La résine plastique peut notamment être produite à partir de déchets de couleur claire.

Par « lumière blanche », on entend une lumière ayant un spectre continu regroupant l'ensemble des longueurs d'onde du domaine visible.
Par « couleur blanche », on entend la couleur associée dans le code RGB aux triplets (r, g, b) tels que r, g, b ≥ 245, voire r, g, b ≥ 250, mieux r, g, b = 255. La différence entre deux composantes du triplet peut notamment être inférieure à 10, voire à 5, mieux à 2. La couleur blanche peut être caractérisée par un test visuel se basant sur les pantones 11-0601, 11-4800 TPX, ou 11-4001 TCX, par exemple.

Comme rappelé précédemment, la couleur blanche est synonyme d'hygiène et de stérilité dans le cadre médical. Elle laisse cependant beaucoup plus facilement voir d'éventuelles impuretés présentes dans le film en matière plastique. L'aspect brillant est également apprécié dans un cadre médical car associé à la notion d'hygiène.

Le film en matière plastique peut être fabriqué au moyen d'une résine plastique recyclée de grade suffisamment élevé pour que le film en matière plastique soit de couleur blanche et/ou ait un aspect brillant.

L'utilisation d'une résine d'un tel grade permet également de contribuer à réduire les impuretés présentes dans le film en matière plastique.

Le plastique recyclé peut être fabriqué au moyen d'une résine plastique recyclée produite à partir de déchets plastiques, lesdits déchets plastiques étant de haute traçabilité, provenant notamment d'un seul fournisseur.

Par « de haute traçabilité », on entend que le ou les fournisseurs des déchets plastiques à la base de la résine peuvent être facilement identifiés et que les déchets plastiques utilisés puissent être suivis dans leur processus de recyclage. Le ou les fournisseurs de déchets plastiques peuvent notamment respecter la norme EN 15343:2007 sur la traçabilité des plastiques recycles.

Le choix d'une résine produite à partir de déchets plastiques de haute traçabilité permet d'assurer une stabilité et une répétabilité du processus de fabrication de la résine, rendant ainsi plus « stable » la matière recyclée.

Un ratio corps étranger/plastique dans les déchets plastiques utilisés pour produire la résine peut être inférieur à 10/90, mieux inférieur à 3/97, encore mieux inférieur à 1/99.

Par « corps étranger », on entend tout élément qui n'est pas du plastique, mais qui peut passer à travers le premier tri des déchets plastiques réalisé. Un corps étranger peut être par exemple une étique d'impression collé sur un film plastique.

Ce ratio renseigne sur la proportion de corps étrangers qu'un lot de déchets provenant d'un fournisseur contient. Moins cette proportion est élevée, plus le tri des déchets sera facile, moins les déchets seront « contaminés » et donc plus le grade de la résine produite à partir de ceux-ci sera élevé.

Le plastique recyclé peut être fabriqué au moyen d'une résine plastique recyclée, la résine plastique recyclée étant produite à partir d'un même type de déchets plastiques.

La résine plastique recyclé peut par exemple être produite à partir des sachets plastiques d'un supermarché donné.

Cette identification d'un fournisseur et d'un type de déchets plastiques contribue à la traçabilité de la résine plastique recyclée. Cela permet de recommander une résine de même qualité, produite avec les mêmes sources plastiques, facilitant de ce fait la stabilité et la répétabilité du processus de fabrication de la résine.

La résine plastique recyclée peut être produite à partir d'un seul déchet plastique, notamment en polyéthylène de faible densité.

La résine plastique recyclée peut être produite à partir du film interne des « *jumbo bags* », ou sacs poufs en français, qui en garantit l'étanchéité. Ce produit est avantageux car il s'agit de plastique vierge non contaminé, et il présente par ailleurs une couleur blanche lorsqu'observé en lumière blanche.

La résine plastique recyclée peut être produite à partir de déchets plastiques autres que des déchets plastiques issus de l'agriculture. Les déchets plastique provenant de l'agriculture sont trop souillés par des éléments externes, comme la terre, la poussière, l'huile les produits chimiques ou les engrais par exemple, pour qu'une résine issue de ses déchets puisse être compatible avec un usage médical, notamment pour qu'elle puisse respecter le règlement MDR. Les déchets plastiques issus de l'agriculture résultent en des résines plastiques dont le grade ne peut dépasser le grade N1A, en utilisant les grades définis par la société CY Plastique listés ci-dessus. Les déchets de l'industrie de la mode et/ou de l'industrie logistique sont à l'origine de résines de grade compris entre AB et NAT%PLUS.

Le plastique PCR peut être fabriqué au moyen d'une résine plastique recyclée produite à partir de déchets plastiques provenant par exemple des circuits logistiques ou des hypermarchés.

Le ou les liens de fermeture peuvent comporter au moins 30% de plastique PIR, mieux au moins 50%, voire au moins 70%, notamment 100% de plastique PIR, et/ou au moins 30% de polyéthylène à haute densité (HDPE), mieux au moins 50%, voire au moins 70%, notamment 100% de HDPE.

La film en matière plastique peut comporter au moins un additif, lequel ou lesquels peuvent être choisi dans la liste suivante : agents glissants, agents antimicrobiens, cette liste n'étant pas limitative. La matière plastique peut comporter entre 1 et 2 % d'additif. L'utilisation d'agents glissants dans des films de polyéthylène permet de réduire le Coefficient de Friction (COF) tout en conservant les propriétés optiques de brillance, de transparence et de clarté.

Le film en matière plastique peut comporter un agent coulissant, de préférence entre 1 et 3% d'agent coulissant, mieux entre 1,5 et 2,5% d'agent coulissant, par exemple 2% d'agent coulissant environ. L'agent coulissant peut être l'agent coulissant Zip-33^{E}, fourni par la société JUSU.

Le ou les liens de fermeture peuvent comporter un agent coulissant, de préférence entre 0,5 et 1,5% d'agent coulissant, mieux entre 0,8 et 1,2% d'agent coulissant, par exemple 1% d'agent coulissant environ. L'agent coulissant peut être l'agent coulissant Zip-33^{E}, fourni par la société JUSU.

Le plastique recyclé est en effet moins glissant que le plastique vierge et ainsi un sac comportant une part importante de plastique recyclé à la fois dans le film de l'enveloppe et dans les liens de fermeture peut présenter des difficultés à faire coulisser les liens. L'utilisation d'agents coulissant dans le film en matière plastique de l'enveloppe et/ou dans le ou les liens de fermeture contribue à réduire ce problème.

Le plastique recyclé peut être fabriqué au moyen d'une résine plastique recyclée produite à partir de déchets plastiques, les déchets plastiques à la base de la résine plastique recyclée étant chauffés au cours de la production de la résine à une température supérieure à 200°C, mieux supérieure à 220°C, notamment d'environ 230°C.

La haute température de chauffe des déchets plastiques permet d'éliminer au moins une partie, voire l'intégralité des possibles pathogènes.

L'invention a encore pour objet un procédé de fabrication d'une résine plastique recyclé comportant l'une au moins des étapes suivantes, voire toutes les étapes suivantes :
a) sélectionner au moins un fournisseur, notamment un unique fournisseur, de déchets plastiques qui ne soient pas issus de l'agriculture,
b) identifier un type de déchets plastiques à sélectionner pour produire la résine, de préférence un type de déchets plastiques peu contaminé, notamment transparent ou de couleur blanche, par exemple le film interne de « *jumbo bags* »,
c) effectuer un ou plusieurs, notamment au moins deux, voire au moins trois, mieux au moins quatre, voire cinq, tris manuels et/ou automatiques des déchets plastiques,
d) chauffer les déchets plastiques triés à une température supérieure à 200°C, mieux supérieure à 220°C, notamment d'environ 230°C.

Le procédé peut comporter une étape e) dans laquelle on réalise des contrôles post-production pour vérifier la qualité de la résine recyclée produite. Ces tests post-production peuvent se baser sur les normes ASTMD1238 et ASTMD792.

Le procédé peut comporter une étape f) dans laquelle on s'assure de la traçabilité de la résine plastique produite en associant à chaque lot produit un numéro de lot.

Le ou les fournisseurs de déchets plastiques peuvent disposer de certificats permettant d'obtenir à partir de ces déchets plastiques une résine plastique certifiée par des organismes indépendants.

La résine plastique recyclée peut être fabriquée dans une usine séparant les lignes de production de résines selon leur grade.

La résine plastique recyclée peut être fabriquée dans une usine ne produisant pas de résines de faible grade, notamment produites à partir de déchets plastiques issus de l'agriculture.

La séparation des lignes de production voire des usines de production des résines permet d'éviter une contamination des résines de grade élevé par les résines de faible grade, par exemple issues de déchets plastiques de l'agriculture.

Les employés de l'usine de fabrication de la résine plastique recyclé peuvent être formés de manière à s'assurer de leur capacité à trier les déchets plastiques et à les catégoriser selon leur niveau de contamination.

Les déchets plastiques à la base de la résine plastique recyclée utilisée peuvent résulter d'un processus de tri comportant plusieurs, notamment au moins deux, voire au moins trois, mieux au moins quatre, voire cinq, tris manuels.

Chaque ligne de production peut être intégralement nettoyée à chaque changement de fournisseur de déchets plastiques et/ou de type de déchets plastiques.

Le fabriquant de la résine plastique recyclé peut disposer d'une certification EUCERPLAST. Le certificat EUCERPLAST se base sur la norme EN15343 relative à la traçabilité des matériaux utilisés pour produire la matière recyclée. Le certificat peut stipuler que le fournisseur a le niveau de traçabilité et donc de tri le plus élevé, à savoir le niveau 1.

Le fabriquant de la résine plastique recyclé peut disposer d'une certification GRS. Cette certification garantit un recyclage respectant des critères environnementaux et sociaux.

Ces certifications permettent d'être en accord avec certaines exigences règlementaires et sont un gage de qualité, notamment pour garantir une stabilité dans la qualité de la résine produite.

Le fabriquant de la résine peut mettre en oeuvre au moins un test évaluant la biocompatibilité de la résine plastique recyclée.

Le fabricant peut mettre en oeuvre un test de caractérisation chimique basé sur la norme ISO 10993-18. Ce test permet de savoir s'il y a des substances notamment cancérigènes, allergisante, irritantes, mutagènes dans la résine.

Le fabricant du sac peut mettre en oeuvre un test de cytotoxicité basé sur la norme ISO 10993-5-2009. Il peut également faire un test de sensibilisation pour s'assurer que le produit ne présente pas de risque lorsqu'en contact avec le patient.

Le film en matière plastique de l'enveloppe peut être produit au moyen d'une machine de soufflage de films munie d'un filtre de maillage compris entre 85 et 120 mesh, notamment d'environ 100 mesh. Le filtre peut être changé régulièrement, notamment toutes les 24 heures, mieux toutes les 12 heures.

L'utilisation d'un filtre de maillage compris entre 85 et 120 mesh qui est changé très régulièrement permet de diminuer encore le niveau d'impuretés dans le film plastique.

Le sac hygiénique peut contenir un tampon absorbant.

Le tampon absorbant peut venir gélifier les fluides corporels (vomissures, urine, excreta) afin de limiter leur propagation. Le tampon absorbant peut être constitué d'un mélange d'ouate et d'une poudre en un polymère super absorbant.

Le tampon absorbant peut être fixé sur une face intérieure de ladite enveloppe, notamment au moyen d'une colle réversible et, de préférence, réutilisable.

L'enveloppe peut comporter des premier et deuxième feuillets identiques, superposés l'un à l'autre et fixés l'un à l'autre le long de bords latéraux droits et gauche et d'un bord de fond, le tampon absorbant étant fixé sur un seul desdits premier et deuxième feuillets.

Selon un autre de ses aspects, l'invention a encore pour objet une bande des sac hygiéniques tels que définis précédemment, chaque sac hygiénique étant fixé par au moins un bord latéral à un sac hygiénique adjacent, lesdits sacs hygiéniques étant notamment séparés les uns des autres par au moins une ligne d'affaiblissement s'étendant le long dudit ou desdits bords latéraux. La bande peut comprendre entre 10 et 30 sacs hygiéniques, notamment 20.

Selon un autre de ses aspects, l'invention a pour objet un ensemble comportant :
- une pluralité de sacs hygiéniques telles que définies précédemment, notamment sous la forme d'une bande telle que définie précédemment, et
- un emballage dans lequel lesdits sacs hygiéniques sont logés.

L'emballage peut être une boite, par exemple une boite en carton. De préférence, l'emballage est une boite en carton 100% recyclé.

L'emballage peut être recyclable. Il peut être certifié « Chaîne de contrôle » FSC, de « *Forest Stewardship council* » en anglais. Ce certificat permet de s'assurer de la traçabilité des matériaux FSC à chaque étape du processus de production, depuis la forêt jusqu'au produit fini, en incluant toutes les étapes successives de traitement, de transformation, de fabrication et de distribution.

Selon un autre de ses aspects, l'invention a enfin pour objet un procédé de fabrication d'un sac hygiénique tel que précédemment défini, comportant les étapes suivantes :
a) confirmer un grade d'une résine plastique recyclée fournie, notamment de polyéthylène à basse densité,
b) préparer un mélange à partir de la résine plastique recyclée et optionnellement d'un mélange-maître agent glissant et/ou d'un agent anti-humidité,
c) remplir avec ledit mélange une machine de soufflage de films, la machine ayant notamment été nettoyée au préalable,
d) disposer un filtre, notamment un filtre neuf, de maillage compris entre 85 et 120 mesh, notamment d'environ 100 mesh, dans la machine de soufflage, le filtre étant notamment destiné à être remplacé régulièrement, notamment au moins toutes les 24 heures, voire toutes les 12 heures,
e) souffler un film avec la machine de soufflage.

Les proportions respectives des éléments du mélange de l'étape b) peuvent être optimisées pour obtenir un film plastique blanc et brillant. Le mélange-maître peut comporter d'autres additifs.

Comme expliqué précédemment, l'utilisation d'agents glissants dans des films de polyéthylène permet de réduire le Coefficient de Friction (COF) tout en conservant les propriétés optiques de brillance, de transparence et de clarté.

La résine plastique recyclée contient plus d'humidité qu'une résine non recyclée en raison des nombreux lavages réalisés au cours de sa production. Cette humidité peut conduire à la formation de bulles lors de l'extrusion du film plastique, ce que la présence d'un agent anti-humidité dans le mélange de l'étape b) contribue à éviter.

Utiliser un filtre de maillage compris entre 85 et 120 mesh, notamment d'environ 100 mesh, en le changeant régulièrement, par exemple toutes les 24 heures ou toutes les 12 heures, permet de réduire les impuretés dans le sac, comme expliqué précédemment.

Le procédé de fabrication peut comporter une étape f) où l'on laisse reposer le film à l'air libre pendant au moins 24 heures. Laisser reposer le film à l'air libre tend à diminuer la mauvaise odeur pouvant accompagner la production du film. Cela contribue également à rendre le film moins collant.

Le procédé de fabrication peut comporter une étape suivant l'étape e) ou l'étape f) précédemment définie, où l'on imprime au moins une information sur le film. La ou les informations imprimées sur le film peuvent comprendre entre autres le nom de la marque, le logo de la marque, le nom du produit, une information sur le volume de déchets pouvant être contenus dans le sac, une mention sur le pourcentage de plastique recyclé. Des paramètres d'impression peuvent être variés à cette étape pour obtenir une impression de qualité.

Le procédé de fabrication peut comporter une étape d'assemblage du sac à partir du film, suivant de préférence une étape d'impression d'au moins une information sur le film.

Le procédé de fabrication peut comporter une étape où on insère un tampon absorbant dans le sac.

L'invention a encore pour objet, selon un autre de ses aspects, un film en matière plastique comportant plus de 30% de plastique recyclé.

Le film en matière plastique peut présenter toutes ou une partie des caractéristiques précédemment décrites.

Le film en matière plastique peut être translucide.

Le film en matière plastique peut être de couleur blanche lorsqu'observé en lumière blanche.

Le film en matière plastique peut être transparent.

Le film en matière plastique peut avoir un aspect brillant.

Selon un autre de ses aspects, l'invention a encore pour objet un sac comportant :
- une enveloppe étanche pourvue d'une ouverture, l'enveloppe étant formée d'un film en matière plastique, le film étant tel que décrit ci-dessus,
- optionnellement, au moins un lien de fermeture de ladite ouverture, le lien de fermeture étant notamment monté coulissant dans un ourlet de l'enveloppe de manière qu'une extraction dudit lien de fermeture hors dudit ourlet réduise ladite ouverture.

Le sac peut présenter toutes ou une partie des caractéristiques précédemment décrites.

Le sac n'est pas nécessairement un sac hygiénique.

Le sac peut être un sac d'usage général, ne nécessitant aucun degré d'hygiène, de stérilité ou autre particulier.

Le sac peut être un sac à usage médical, en particulier stérilisé.

Le sac peut être un sac hygiénique, notamment à usage médical, en particulier stérilisé.

Le sac peut être adapté au stockage et/ou au transport d'aliments en toute sécurité.

Le sac peut être à usage domestique ou industriel.

### Brève description des dessins

L'invention pourra être mieux comprise à la lecture de la description qui va suivre, d'exemples de mise en oeuvre non limitatifs de celle-ci, et à l'examen du dessin annexé, sur lequel :
[Fig 1] La figure 1 représente un sac hygiénique selon l'invention.
[Fig 2] La figure 2 illustre les principales étapes de fabrication d'un sac hygiénique selon l'invention.

### Description détaillée

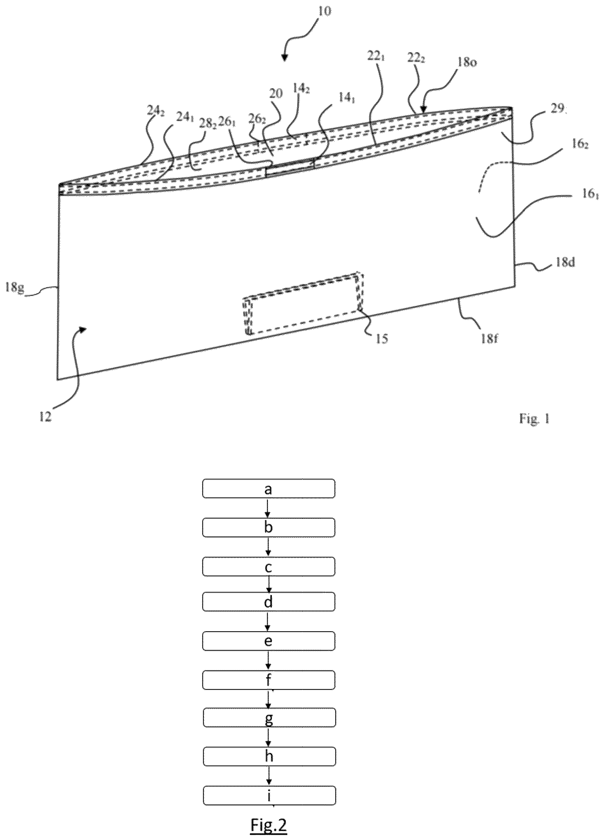

On a illustré à la figure 1 un sac hygiénique 10 à usage unique destiné à récupérer les déchets, notamment au moins en partie liquides, d'un patient. Le sac hygiénique 10 est un protège seau et/ou un protège bassin. Il peut contenir un volume de déchets par exemple de 450 mL.

Le sac hygiénique 10 comporte une enveloppe 12 pourvue d'une ouverture 20 et de premier et deuxième liens de fermeture 14₁ et 14₂. L'enveloppe 12, généralement de forme rectangulaire quand le sac est à plat, comporte des premier et deuxième feuillets 16₁ et 16₂, respectivement, de mêmes dimensions, superposés et fixés l'un à l'autre, de manière étanche, le long d'un bord latéral droit 18d, d'un bord latéral gauche 18g et d'un bord de fond 18f reliant les extrémités inférieures des bords latéraux droit et gauche. Les premier et deuxième feuillets peuvent résulter du pliage d'une feuille le long du bord de fond 18f. L'étanchéité des bords latéraux droit et gauche peut être obtenue par exemple par soudure. L'enveloppe est dépourvue de soufflet. Elle est aussi dépourvue de bretelles de préhension.

L'ouverture 20 du sac hygiénique est définie par un bord d'ouverture 18o constitué des premier et deuxième bords libres 22₁ et 22₂ des premier et deuxième feuillets, respectivement. Le long des premier et deuxième bords libres, les premier et deuxième feuillets définissent des premier et deuxième ourlets 24₁ et 24₂, respectivement, interrompus par des première et deuxième fenêtres 26₁ et 26₂, respectivement. Les premier et deuxième liens de fermeture sont montés coulissants dans les premier et deuxième ourlets, respectivement. Ils sont accessibles à travers les première et deuxième fenêtres, respectivement. Leurs extrémités droite et gauche sont fixées sur les bords droit 18d et gauche 18g de l'enveloppe, respectivement, de sorte que leur extraction par les première et deuxième fenêtres, respectivement, ou « serrage », provoque une contraction des premier et deuxième bords libres 22₁ et 22₂, et donc l'étranglement de l'ouverture 20. Les premier et deuxième feuillets 16₁ et 16₂ définissent des première et deuxième faces intérieures 28₁ et 28₂.

Les liens de fermeture 14₁ et 14₂ sont des rubans. Ces rubans sont d'une largeur supérieure à 20 mm, notamment d'environ 25 mm. Les liens de fermeture 14₁ et 14₂ peuvent comporter au moins 30% de plastique PIR, notamment 100% de HDPE dans l'exemple décrit.

L'enveloppe 12 comporte un tampon absorbant 15. Ce tampon 15 est constitué d'un mélange d'ouate et d'une poudre en un polymère super absorbant. Le volume du tampon absorbant est adapté pour absorber les déchets liquides introduits dans l'enveloppe par le patient.

Le tampon absorbant 15 est fixé sur au moins une des faces intérieures de l'enveloppe, de préférence sur la seule deuxième face intérieure 28₂. Le tampon 15 est fixé au moyen d'une colle réversible, c'est-à-dire choisie de manière à autoriser un décollement du tampon absorbant 15 et/ou du deuxième feuillet sans détérioration du tampon absorbant 15 et/ou du deuxième feuillet, respectivement.

L'enveloppe 12 est formée d'un film en matière plastique 29 comportant plus de 30% de plastique recyclé. Ce film en matière plastique est d'une épaisseur inférieure à 40 microns, notamment d'environ 32 microns. Le plastique recyclé est fabriqué à partir d'une résine plastique recyclée de l'entreprise CY plastique de grade supérieur ou égal au grade NAT% PLUS. La résine plastique recyclée utilisée présente une densité d'environ 0,9169g/cm³ et d'indice de fluidité à chaud d'environ 1,60g/10 min. Le film en matière plastique 29 de l'enveloppe 12 peut comporter plus de 30% de plastique recyclé post-consommation (PCR), notamment dans l'exemple décrit 100% de plastique PCR.

Le film en matière 29 peut comporter au moins 30% de polyéthylène à basse densité (LDPE), notamment dans l'exemple décrit 100% de polyéthylène à basse densité.

La résine plastique recyclée est configurée pour que le film en matière plastique 29 soit de couleur blanche lorsqu'observé en lumière blanche et ait un aspect brillant.

Le film en plastique recyclé 29 est fabriqué au moyen d'une résine plastique recyclée produite à partir de déchets plastiques provenant d'un seul fournisseur. La résine plastique recyclée est produite à partir d'un même type de déchets plastiques. Il s'agit du film interne des « jumbo bags » ou sacs poufs en français.La figure 2 illustre schématiquement un procédé pouvant être mis en oeuvre pour fabriquer un sac hygiénique 10 selon l'invention.

Dans une première étape a), on confirme un grade d'une résine plastique recyclée, notamment de polyéthylène à basse densité fournie.

A l'étape b), on prépare un mélange à partir de la résine plastique recyclée et optionnellement d'un mélange-maître agent glissant. Les proportions respectives des éléments de ce mélange sont optimisées pour obtenir un film plastique 29 blanc et brillant.

Dans une étape c), on remplit avec ledit mélange une machine de soufflage de films, la machine ayant notamment été nettoyée au préalable.

L'étape d) comprend la disposition d'un filtre, notamment un filtre neuf, de maillage compris entre 85 et 120 mesh, notamment d'environ 100 mesh, dans la machine de soufflage, destiné à être remplacé notamment au moins toutes les 24 heures, voire toutes les 12 heures.

L'étape e) est l'étape de soufflage du film avec la machine de soufflage.

Eventuellement une étape f) complète ce procédé, dans laquelle on laisse reposer le film à l'air libre pendant au moins 24 heures.

Le procédé peut également éventuellement comporter une étape g) suivant ces étapes où l'on imprime au moins une information sur le film, notamment une information sur la marque et le logo du fabricant du sac hygiénique 10 ou une information sur le volume de déchets contenus dans le sac 10.

Une étape h) d'assemblage du sac 10 à partir du film plastique 29 peut être inclue dans ce procédé. Enfin, le procédé peut comporter l'étape i) d'insertion du tampon absorbant 15 dans le sac 10.

L'invention n'est pas limitée aux exemples décrits.

Le sac hygiénique 10 peut être un sac urinal ou un sac vomitoire. Dans ce mode de réalisation, le ou les liens de fermeture peuvent être des cordelettes.

Dans un mode de réalisation, le film en matière plastique 29 de l'enveloppe 12 peut comporter plus de 30% de plastique recyclé post-industriel (PIR), mieux au moins 40% de plastique PIR, mieux au moins 50% de plastique PIR, encore mieux au moins 60% de plastique PIR, encore mieux au moins 70% de plastique PIR, encore mieux au moins 80% de plastique PIR, encore mieux au moins 90% de plastique PIR, notamment 100% de plastique PIR.

Le tampon absorbant 15 peut aussi par exemple être logé dans l'enveloppe, sans y être fixé.

## Revendications

1. Sac hygiénique (10) à usage médical destiné à la récupération de déchets produits par un patient, ledit sac hygiénique (10) comportant :
- une enveloppe (12) étanche pourvue d'une ouverture (20), l'enveloppe (12) étant formée d'un film en matière plastique (29),
- optionnellement, au moins un lien de fermeture (14₁, 14₂) de ladite ouverture, le lien de fermeture (14₁, 14₂) étant notamment monté coulissant dans un ourlet (24₁, 24₂) de l'enveloppe (12) de manière qu'une extraction dudit lien de fermeture (14₁, 14₂) hors dudit ourlet (24₁, 24₂) réduise ladite ouverture (20),
**caractérisé en ce que** le film en matière plastique (29) de l'enveloppe comporte plus de 30% de plastique recyclé.

2. Sac hygiénique (10) selon la revendication précédente, ledit plastique recyclé étant fabriqué à partir d'une résine plastique recyclée de grade suffisamment élevé pour que l'indice de fluidité à chaud de la résine soit supérieur ou égal à 1,40 g/10min, mieux supérieur ou égal à 1,50g/10min, encore mieux supérieur ou égal à 1,60g/10 min.

3. Sac hygiénique (10) selon l'une des deux revendications précédentes, ledit plastique recyclé étant fabriqué à partir d'une résine plastique recyclée de grade suffisamment élevé pour que la densité de la résine soit supérieure à 0,9g/cm³.

4. Sac hygiénique (10) selon l'une des revendications précédentes, le film en matière plastique (29) de l'enveloppe (12) comportant plus de 30% de plastique recyclé post-consommation (PCR), mieux au moins 40% de plastique PCR, mieux au moins 50% de plastique PCR, encore mieux au moins 60% de plastique PCR, encore mieux au moins 70% de plastique PCR, encore mieux au moins 80% de plastique PCR, encore mieux au moins 90% de plastique PCR, notamment 100% de plastique PCR.

5. Sac hygiénique (10) selon l'une des revendications précédentes, le film en matière plastique (29) de l'enveloppe (12) comportant plus de 30% de plastique recyclé post-industriel (PIR), mieux au moins 40% de plastique PIR, mieux au moins 50% de plastique PIR, encore mieux au moins 60% de plastique PIR, encore mieux au moins 70% de plastique PIR, encore mieux au moins 80% de plastique PIR, encore mieux au moins 90% de plastique PIR, notamment 100% de plastique PIR.

6. Sac hygiénique (10) selon l'une quelconque des revendications précédentes, le film en matière plastique (29) comportant au moins 30% de polyéthylène à basse densité (LDPE), mieux au moins 50%, voire au moins 70%, notamment 100% de polyéthylène à basse densité.

7. Sac hygiénique (10) selon tout rattachement à la revendication 2 ou 3, ladite résine plastique recyclée étant configurée pour que le film en matière plastique (29) soit de couleur blanche lorsqu'observé en lumière blanche et/ou ait un aspect brillant.

8. Sac hygiénique (10) selon l'une quelconque des revendications précédentes, le plastique recyclé étant fabriqué au moyen d'une résine plastique recyclée produite à partir de déchets plastiques, lesdits déchets plastiques étant de haute traçabilité, provenant notamment d'un seul fournisseur.

9. Sac hygiénique (10) selon l'une quelconque des revendications précédentes, le ou les liens de fermeture (14₁, 14₂) comportant au moins 30% de plastique PIR, mieux au moins 50%, voire au moins 70%, notamment 100% de plastique PIR, et/ou au moins 30% de polyéthylène à haute densité (HDPE), mieux au moins 50%, voire au moins 70%, notamment 100% de HDPE.

10. Sac hygiénique (10) selon l'une quelconque des revendications précédentes, le film en matière plastique (29) comportant au moins un additif choisi dans la liste suivante : agents glissants ou agents antimicrobiens, cette liste n'étant pas limitative.

11. Sac hygiénique (10) selon l'une quelconque des revendications précédentes, le plastique recyclé étant fabriqué au moyen d'une résine plastique recyclée produite à partir de déchets plastiques, les déchets plastiques à la base de la résine plastique recyclée étant chauffés au cours de la production de la résine à une température supérieure à 200°C, mieux supérieure à 220°C, notamment d'environ 230°C.

12. Sac hygiénique (10) selon l'une quelconque des revendications précédentes, le film en matière plastique (29) de l'enveloppe (12) étant produit au moyen d'une machine de soufflage de films munie d'un filtre de maillage compris entre 85 et 120 mesh, notamment d'environ 100 mesh, le filtre étant notamment changé toutes les 24 heures, mieux toutes les 12 heures.

13. Sac hygiénique (10) selon l'une quelconque des revendications précédentes, comportant un tampon absorbant (15).

14. Sac hygiénique (10) selon la revendication précédente, le tampon absorbant (15) étant fixé sur une face intérieure (28₁, 28₂) de ladite enveloppe (12), notamment au moyen d'une colle réversible et, de préférence, réutilisable.

15. Sac hygiénique (10) selon l'une des deux revendications précédentes, l'enveloppe (12) comportant des premier et deuxième feuillets (16₁, 6₂) identiques, superposés l'un à l'autre et fixés l'un à l'autre le long de bords latéraux droits (18d) et gauche (18g) et d'un bord de fond (18f), le tampon absorbant (15) étant fixé sur un seul desdits premier et deuxième feuillets.

16. Bande de sacs hygiéniques (10) selon l'une quelconque des revendications précédentes, chaque sac hygiénique (10) étant fixé par au moins un bord latéral (18d, 18g) à un sac hygiénique adjacent (10), lesdits sacs hygiéniques (10) étant notamment séparés les uns des autres par au moins une ligne d'affaiblissement s'étendant le long dudit ou desdits bords latéraux.

17. Ensemble comportant :
- une pluralité de sacs hygiéniques (10) selon l'une quelconque des revendications 1 à 15, notamment sous la forme d'une bande selon la revendication 16, et
- un emballage dans lequel lesdits sacs hygiéniques sont logés.

18. Procédé de fabrication d'un sac hygiénique (10) selon l'une quelconque des revendications là 15, comportant les étapes suivantes :
a) confirmer un grade d'une résine plastique recyclée fournie, notamment de polyéthylène à basse densité,
b) préparer un mélange à partir de la résine plastique recyclée et optionnellement d'un mélange-maître agent glissant et/ou d'un agent anti-humidité,
c) remplir avec ledit mélange une machine de soufflage de films, la machine ayant notamment été nettoyée au préalable,
d) disposer un filtre, notamment un filtre neuf, de maillage compris entre 85 et 120 mesh, notamment d'environ 100 mesh, dans la machine de soufflage, le filtre étant destiné à être remplacé notamment au moins toutes les 24 heures, voire toutes les 12 heures,
e) souffler un film (29) avec la machine de soufflage.
